# EUROPEAN PATENT SPECIFICATION

(11) **EP 0 912 245 B1**
(45) Date of publication and mention of the grant of the patent: **20.02.2002**
(21) Application number: 97931954.8
(22) Date of filing: 16.07.1997
(51) Int. Cl.: B01L 3/00, A61B 10/00, G01N 33/52

(54) **NEW TEST DEVICE FOR MASS SCREENING**
VORRICHTUNG ZU REIHENUNTERSUCHUNGEN
NOUVEAU DISPOSITIF D'ESSAI POUR CRIBLAGE DE MASSE

(30) Priority: 17.07.1996 GB 9614851
(43) Date of publication of application: 06.05.1999
(73) Proprietor: Cunningham, Robert William, Washington, Tyne & Wear NE38 8AE (GB)
(72) Inventor: Cunningham, Robert William, Washington, Tyne & Wear NE38 8AE (GB)
(74) Representative: Allen, Philippa Margaret
(86) International application number: GB9701939
(87) International publication number: WO9802249

(56) References cited:
- EP-A- 0 583 078
- WO-A-93/11434
- US-A- 2 129 754
- US-A- 3 418 083
- US-A- 4 622 207
- US-A- 4 846 182
- US-A- 5 409 664
- US-A- 5 520 041

## Description

The invention relates to a test device for use particularly but not exclusively, in automated testing apparatus and is particularly advantageous for the handling, drying, storage, transport or the like and subsequent analysis of fluid samples or the like.

Mass screening of specific subsections of the population for a range of diseases and conditions to which members of that subsection are susceptible, is a well known early warning health care measure. The aim of such screening is, principally, not to diagnose those people with the disease or condition but, rather, to reject the vast majority of the people who are clearly normal. Those who show abnormal results can then be further examined to provide a diagnosis.

A typical procedure for testing a particular cross section of the population would involve the taking of a sample of blood, urine or saliva, for example. This is usually done in a clinic, surgery or hospital where there are specialised staff ready to process, package and label such samples for transportation to a specialised laboratory for further analysis.

There are many diseases and conditions which can be screened in this way. For example; osteoporosis in post-menopausal women; in-born errors of metabolism such as phenylketonuria; metabolic disorders such as thyroid disease; clinical conditions such as neuroblastoma in children; drug and/or alcohol abuse; chromosomal abnormalities; infectious diseases; DNA profiling and the like. However, current methods and procedures for mass screening and subsequent analysis are particularly time consuming for the specialised laboratory and are, as a result, expensive.

The time consuming nature of such methods, is apparent from conventional neo-natal screening of babies. Usually, a nurse will prick the heel of, typically, a five day old infant with a needle in order to obtain a blood sample. The blood is deposited in several, usually four, predetermined locations on an absorbent test card. Each location is usually defined by a printed circle on the test card. The card also contains space for identifying details of the child under examination etc.

In order that later testing in the laboratory is carried out on approximately the same quantity of blood from each child, the nurse will normally try and ensure that within each printed circle of the card there is evenly and completely impregnated a sample of blood. This is not always possible to achieve as the child may, for example, wriggle against the paper thereby smearing blood over a greater area so that a second application is required.

Once the blood has been collected on to the absorbent test card, it must be dried prior to insertion in an envelope, or other suitable carrying means, for subsequent postage or transportation to an analysis laboratory. Air drying of samples is the method usually adopted. Typically, the nurse will move the card backwards and forwards through the air for a few minutes to facilitate drying. It is not uncommon, however, for incompletely dried samples to be placed within an envelope for transportation to the laboratory. This is to be expected given that complete drying in static air of blood samples absorbed on material, such as filter paper, can take up to four hours.

The next stage of the procedure takes place in the analysis laboratory. The technician manually punches a hole in the blood impregnated portion of the test card, judging by eye the optimum position from which to take a sample. Typically a 3mm circle of card will be removed. This is then placed in a test tube containing a reagent into which the blood passes. The sample of card is removed from the test tube leaving the blood sample ready to be further processed.

EP-A-0.583.078 discloses a method and apparatus for handling samples and a sample collection system, wherein a blood sample is absorbed into a substrate such as filter paper.

Alternatively, instead of absorbent material such as filter paper, a hydrophobic membrane is used as a supportive material on which blood samples are collected. Such a sample is dried as before and sent to a laboratory for analysis. Once in the laboratory, the sample may be removed by the passing of water, or other suitable solvent, over the hydrophobic membrane so that the sample is simply washed into, for example, a test tube.

Ideally, the quantity of blood obtained for each test from each child is approximately constant. Hence the use of a standard 3mm card sample. Typically, two tests are carried out on samples from each child, one for phenylketonuria and the second for thyroid disease. The absorbent test card, containing any remaining samples, is stored in case the need for repeat or further tests should arise.

Mass screening for osteoporosis in post-menopausal women is similarly time consuming in that, a mid stream urine sample obtained from the patient in, for example, a bottle must be labelled and stored and later transported to a laboratory for subsequent analysis. Samples contained in bottles require specialised packing arrangements in order that these can be transported safely and securely. This sample management is an essential though repetitive and menial task that is usually carried out by professional staff. At present, such samples can be obtained by patients in the privacy of their own homes, but these are then later taken, usually by hand, to a clinic for further processing.

A system for the collection of a saliva sample by a patient at home has already been developed. This system incorporates a piece of absorbent paper fixed on the end of a holding stick. The paper is placed in the mouth using the holding stick. Following absorbtion of saliva, the paper is placed in a buffer solution contained in a small sealable bottle. The entire arrangement of paper, stick, buffer solution and bottle are then taken to a laboratory. In this way, the integrity of a fluid sample obtained by a patient at home is preserved during storage and transport of the sample.

There are several problems to the use of such procedures for the mass screening of the population.

Firstly, if a test card is used, there is a risk of contamination of the samples by contact with external objects, in particular, surfaces. This can occur when the test card is completely dry, but it is especially a problem when a damp card is placed in, for example, an envelope. It is impractical for a nurse in the field to wait up to four hours for the test card to be completely dry before handling it further.

The transportation of bottled samples poses particular problems in that bottles have a 3-D shape and are less easy to label and send by post. In addition, there is always an inherent risk of leakage and/or breakage which not only results in the loss of the sample but can prove hazardous. Furthermore, samples stored in liquid form are inherently susceptible to deterioration and contamination, particularly if storage conditions, such as temperature etc., are not adequately controlled. Storage of dry samples, however, effectively freezes said samples at a point in time such that little or no deterioration occurs.

Secondly, the manipulations to be carried out by professional staff such as a nurse or doctor in the clinic in labelling and packaging such samples, and a technician in the laboratory in processing such samples, are of a highly repetitive and time consuming nature.

Thirdly, the value judgement of the laboratory technician in determining the optimum place on a test card to punch and so obtain samples, is open to error, particularly because of the repetitive nature of the work.

Fourthly, the cost of mass screening lies predominantly in the time taken to obtain, process, transport and analyse each sample from each patient. Currently, professional staff are involved in each of these stages.

The cost of mass screening could be significantly reduced, if people could, in the privacy of their homes, provide samples of, for example, blood, urine or saliva or any other body fluid which can be safely stored and/or sent to a central analysis centre without the risk of contamination and without the need for the involvement of professional staff.

Indeed, the cost of mass screening of fluid samples from a variety of sources could be reduced if such samples could be transported and analysed in the above manner. Examples of sources from which fluid samples could be taken include natural water courses, household or industrial water tanks and pipes, household pets and farm animals.

The invention of the device has elegantly and inventively overcome many of the problems associated with the prior art by providing a user friendly, effective test device comprising a substrate suitably adapted so as to provide aperture(s) wherein said apertures are suitably adapted so as to support selected supportive material so that fluid sample(s) can be efficiently dispensed onto said supportive material located within said aperture(s). Furthermore, said supportive material is further adapted so as to provide suitable guide means for checking the adequacy of the sample collected. Subsequently said test device can be placed in a suitable pouch or the like, wherein said pouch is adapted so as to provide dessicance properties and thus to dry a fluid sample more efficiently. Eventually said test device is presented to an automated analyzer for processing or the like.

In its broadest aspect the invention provides a mass screening test device capable of collecting multiple body fluid samples for subsequent or in situ analysis.

It is therefore an object of the invention to provide a test device and carrying means or envelope to facilitate the taking of samples and the subsequent postage or transportation to a laboratory, which test device, in addition, is further adapted to be used in conjunction with automated testing apparatus.

It is a yet further object of the invention to provide a means for confirming the adequacy of the sample collected.

It is yet a further object of the invention to provide a reliable test device for use in a variety of diagnostic applications.

It is a yet further object of the invention to provide a test device for use with any body fluid and/or matter such as blood, urine, saliva, faeces or the like.

In a first aspect of the invention there is provided a test device for use in automated testing apparatus comprising the features of claim 1. The positioning of a sample to be tested on said supportive material can thus be recognized by automated testing apparatus and said sample to be tested can be optionally removed therefrom.

In a preferred embodiment of the invention, the supportive material is spaced from an outermost surface of said substrate. Ideally, the supportive material is sandwiched between two substrates whereby, the supportive material is spaced from said outermost surface by the thickness of one substrate.

In a further preferred embodiment of the invention, there is provided a plurality of evenly spaced first indentations or apertures.

Ideally, the substrate or substrates of said test device is or are adapted to be easily manipulated by automated testing apparatus. Preferably, the test device comprises a holding means, such as ridges or holes, whereby the handling of the test device by automated apparatus is facilitated. Preferably, said first aperture is a throughbore.

In a yet further preferred embodiment of the invention at least a part of at least one surface of said support material is provided with a suitable hydrophobic material, ideally latex or wax or the like, configured so as to provide the guide means, which typically comprises a line of said hydrophobic material, wherein said configuration is such that ideally a circular portion and a channel portion is defined, wherein following application of a fluid sample, fluid is allowed to permeate to an edge of said circular portion and excess fluid is directed along said channel portion.

In a yet further preferred embodiment of the invention said substrate is provided with at least one second aperture or indentation suitably sized and shaped and positioned, with respect to said first aperture, so as to be aligned with said channel portion of said guide means.

In a yet further preferred embodiment of the invention there is provided an indicator means suitably positioned with respect to said second aperture indentation, ideally said indicator means is associated with or impregnated with or cross-linked to or coated onto at least a part of a least one surface said supportive material.

In a yet further preferred embodiment of the invention the diameter of said guide means circular portion is greater than the diameter of the first aperture or indentation diameter and ideally is greater in diameter in the region of 1-5mm and most ideally 2-3mm.

In a yet further preferred embodiment of the invention, the supportive material or at least a part of the surface of the supportive material is adapted to efficiently and, ideally, quickly distribute a fluid sample into at least a part of the supportive material or across at least a part of the surface of the supportive material. This can be achieved by modifying physically or chemically the nature of the surface. Preferably, the supportive material is absorbent in nature, such as, filter paper. Alternatively, the supportive material may be a hydrophobic membrane.

In a yet further preferred embodiment of the invention said supportive material or at least part of the surface of said supportive material is associated or impregnated with or cross-linked to or comprises or is coated with a suitable selected material whereby a fluid sample can react directly with said material in a colourmetric and/or fluorometric and/or luminometric and/or radiometric manner whereby fluid samples may be analysed at the point in time of collection.

In a yet further preferred embodiment of the invention said test device is provided with an identification means.

In a yet further aspect of the invention, there is provided a pouch for receiving a test device, according to the invention.

Ideally, the pouch comprises a desiccant layer. Preferably, the desiccant layer comprises at least a part of at least one surface, ideally the inner surface, of the pouch.

In a further preferred embodiment of the invention, the pouch comprising a desiccant layer, is so sized and shaped so that when a test device is inserted into the pouch, the supportive material contained in the test device is opposite or adjacent the desiccant layer. Preferably, the desiccant layer comprises silica gel. Furthermore, the pouch, at least a part of its outer surface, may comprise impervious material.

In a yet further aspect of the invention, there is provided a test kit comprising at least one test device according to the invention and at least one pouch according to the invention.

In a yet further aspect of the invention, there is provided a test kit comprising a test device according to the invention, a pouch and a means for obtaining a sample. Ideally, the means for obtaining a sample comprises a lance or blade, preferably automatic, if a blood sample is required, a pipette if a saliva sample is required, and/or, possibly, a container if a urine and/or stool sample is required. In addition, the test kit may comprise insructions and/or a bar code for identifying purposes.

Ideally, the bar code is used to indicate the identity and origin of each individual test device, the type of test to be carried out and/or the particular shape of the test device whereby automated testing apparatus can be automatically reconfigured following reading of the bar code to accommodate test devices of a variety of shapes and for a variety of tests.

In a yet further aspect of the invention there is provided a method for confirming the adequacy of a collected fluid sample comprising;
i) providing a test device comprising the features of claim 1,
ii) placing said fluid sample on said deposition portion and allowing said fluid sample to fill and/or permeate into said channel portion;
iii) collecting sufficient fluid of said sample so that said sample passes over an indicator means in or associated with said channel portion;
iv) assessing said collected fluid sample by visualisation of said indicator means and/or by automated machine analysis of said indicator means.

Particular embodiments of the invention will now be described with reference to the accompanying drawings and by way of example only.

In the accompanying drawings:

Figure 1a shows a plan view of a test device illustrative of certain features useful for understanding the invention.

Figure 1b shows a side sectional view of a test device illustrative of certain features useful for understanding the invention.

Figure 1c shows a plan view of a section of supportive material.

Figure 1d shows a plan view of an alternative test device in accordance with the invention.

Figure 2a shows a plan view of an alternative test device in accordance with the invention.

Figure 2b shows a side view of an alternative test device in accordance with the invention

Figure 3 shows a plan view of an alternative test device in accordance with the the invention.

Figure 4a shows a plan view of an alternative test device in accordance with the invention.

Figure 4b shows a side view of an alternative test device in accordance with the invention.

Figure 5a shows a plan view of an alternative test device in accordance with the invention.

Figure 5b shows a side view of an alternative test device in accordance with the invention.

Figure 6 shows a perspective view of a pouch and a test device illustrative of certain features useful for understanding the invention.

Figure 7 shows a sectional view of a pouch illustrative of certain features useful for understanding the invention.

Figure 8 shows a sectional view of an alternative pouch illustrative of certain features useful for understanding the invention.

Particular embodiments of the invention will now be described.

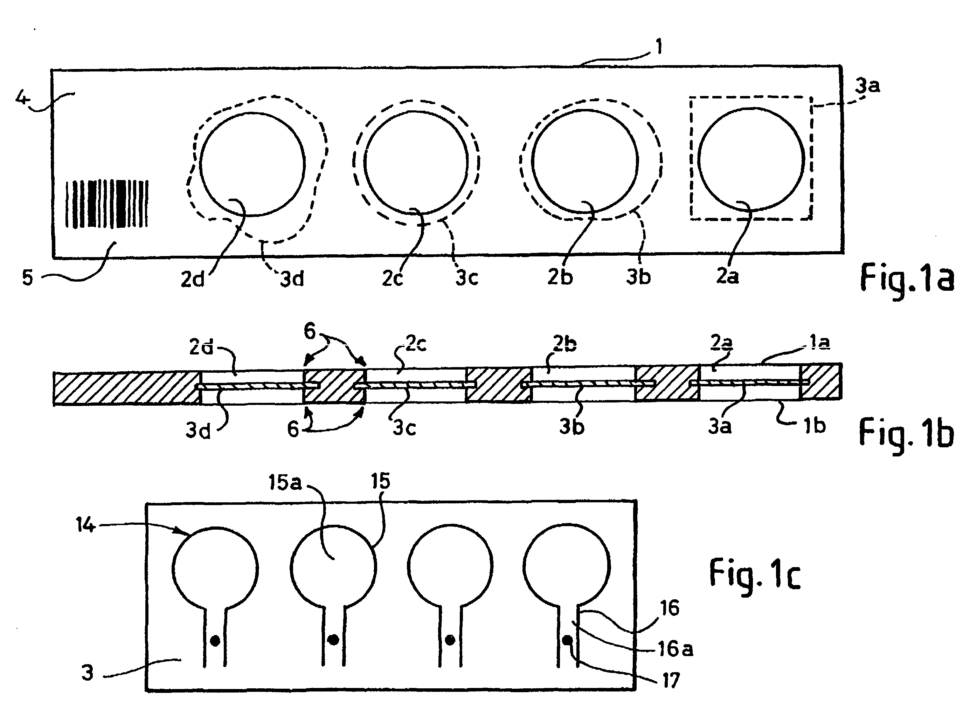

Referring firstly to figure 1a, rectangular substrate 1 is clearly shown. Substrate 1 comprises four first apertures 2a, 2b, 2c and 2d of a particular size, shape and location. Substrate 1 may be made of any suitable non-absorbent material such as plastic. In this particular embodiment, the first apertures, generally labelled 2, are circular. A region for noting the identifying particulars of a patient by means of a label or otherwise is provided towards one end of the substrate 1. Also provided is a region 5 comprising a bar code. The bar code can be used to include several pieces of information for later use by automated testing apparatus. This information may comprise, for example, the batch number or identifying number of each individual test device, the type of test device used ie its particular shape or geometric orientation and/or a specific test or tests that are to be undertaken on the samples located within the test device. The use of a bar code containing such information facilitates the use of such test devices in automated analytical apparatus.

Spanning first apertures 2 are correspondingly numbered sections of supportive material labelled 3a, 3b, 3c and 3d, which supportive material sections may be a variety of shapes as shown clearly in figure 1a. For example, supportive material section 3a is generally square whereas supportive material section 3c is generally circular.

The supportive material located within each aperture may be absorbent or adapted to facilitate wetting of a fluid sample to the surface of the material. This can be achieved in a number of ways, for example, the use of absorbent materials, such as filter paper. Alternatively, a surface wetting agent may be applied to a part of the exposed surface of each supportive material section.

Turning now to figure 1b, first apertures 2 are clearly shown to be throughbores. Furthermore, substrate 1 is clearly shown to comprise an upper portion 1a and a lower portion 1b which are aligned and sandwiched together so as to provide apertures 2. Furthermore, portions 1a and 1b enclose and support the supportive material sections 3a, 3b, 3c and 3d, so that supportive material spans each of the first apertures 2.

Alternatively, this may be achieved by a piece of supportive material 3 (not shown) spanning all the first apertures in substrate 1.

As can be seen clearly from figure 1b, supportive materials sections 3a, 3b, 3c and 3d are suspended tightly across first apertures 2 (similarly a single piece of supportive material 3 may be so arranged), so as to safeguard against sections 3a, 3b, 3c and 3d (or material 3) making contact with adjacent objects. In this way, contamination of samples carried on sections 3a, 3b, 3c and 3d or (supportive material 3) is avoided. Upper or lower outermost edges 6 of first aperture 2 may be adapted to allow easy access of a finger, or other sample supporting means, to the supportive materials sections 3a, 3b, 3c and 3d (or material 3).

Referring now to Figure 1c, this Figure shows a section of support material (3) whereby at least one guide means (14) is provided on at least one surface of said support material wherein said guide means is configured so as to provide a circular portion (15) and a channel portion (16). The delineation of said guide means (14) is provided by a hydrophobic material such as latex or wax and the configuration provides for a fluid sample to permeate across the region (15a) and upon reaching an edge of circular portion (15) fluid to be directed along region (16a) of channel portion (16) so as to pass over indicator means (17). Indicator means (17) comprises an indicator printed onto the surface of said supportive material so that fluid samples passing thereover can react with said indicator means so as to elicit a change of colour, in the instance of said indicator means comprising an anhydrous copper sort or the like. Activation of said indicator means thereby assures the adequacy of the fluid sample collected. The size and shape of the guide means may be varied according to a user's requirement.

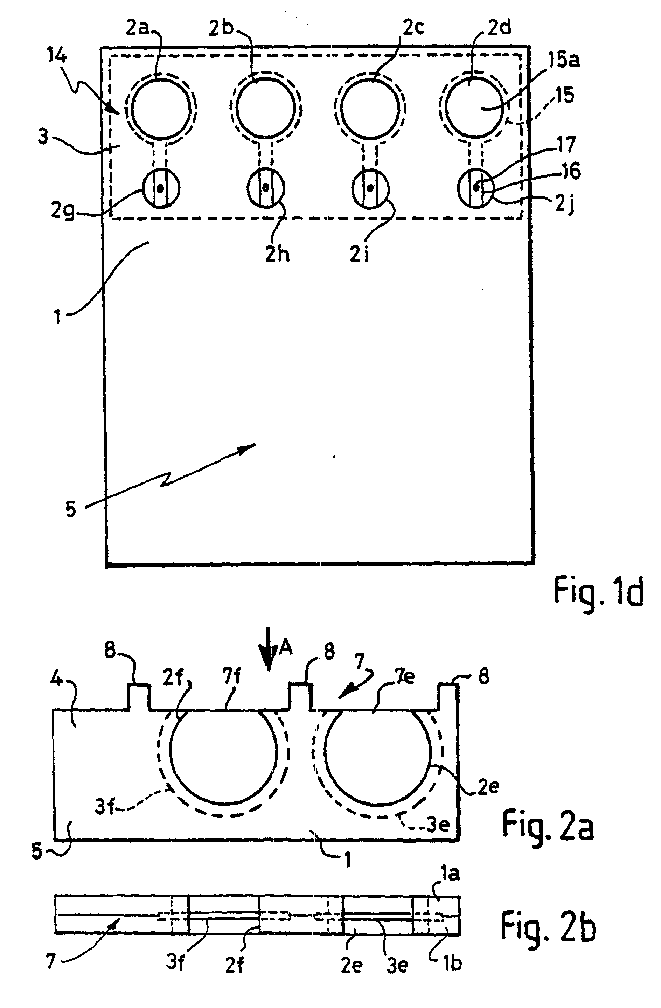

Referring now to Figure 1d, this Figure shows supportive material (3) as depicted in Figure 1c placed rear to the of the substrate (1). Additionally provided on substrate (1) is a region (5) comprising a bar code. First apertures 2a, 2b,2c, 2d of substrate (1) are suitably positioned over guide means (14) so as to be aligned with the circular portion (15) of said guide means (14) so as to accommodate fluid sample collection. Channel portion (16) of guide means (14) is aligned with second apertures 2g, 2h, 2j, 2i of substrate (1) so that the indicator means (17) are suitably positioned so as to be observed or analysed in either a manual or an automatic manner thereby ensuring the adequacy of sample collection to be directly and/or indirectly observed. In operation, fluid samples are collected on area (15a) and permeate outwards to the edge of circular portion (15a) and excess fluid is directed along channel (16) so as to pass over indicator means (17) whereby fluid sample collection maybe ensured and/or checked by appropriate activation of said indicator means.

Figures 2a and 2b show an alternative embodiment of a test device. Corresponding labels refer to corresponding portions of the test device as previously described. In this embodiment, indentations 2e and 2f are located in substrate 1 such that side 7 of substrate 1 is apertured or cut away at the location of indentations 2e and 2f. Corresponding supportive material 3e and 3f respectively is sandwiched between portions 1a and 1b such that the sides of indentations 2e and 2f substantially surround the supportive material sections 3e and 3f. Edges 7e and 7f of respective supportive material sections 3e and 3f are exposed adjacent to side 7 of substrate 1.

Exposure of edges 7e and 7f aid the application of, for example, a finger on which there is a blood spot, to supportive material 3e and 3f in the general direction A. Furthermore, the addition of stops 8 reduce the risk of contacting edges 7e and 7f with external surfaces and, therefore, reduce the risk of contamination of samples contained on 3e and 3f.

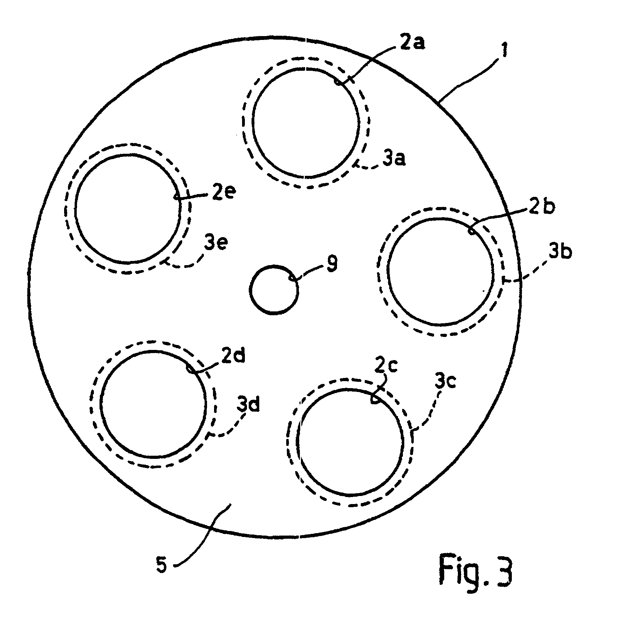

Referring now to figure 3, an alternative embodiment of a test device is shown in which a plurality of first apertures 2 are disposed about a generally circular substrate 1. Means may be provided on substrate 1 for the handling of the test device in automated test apparatus. In this embodiment, a hole 9 is provided for this purpose.

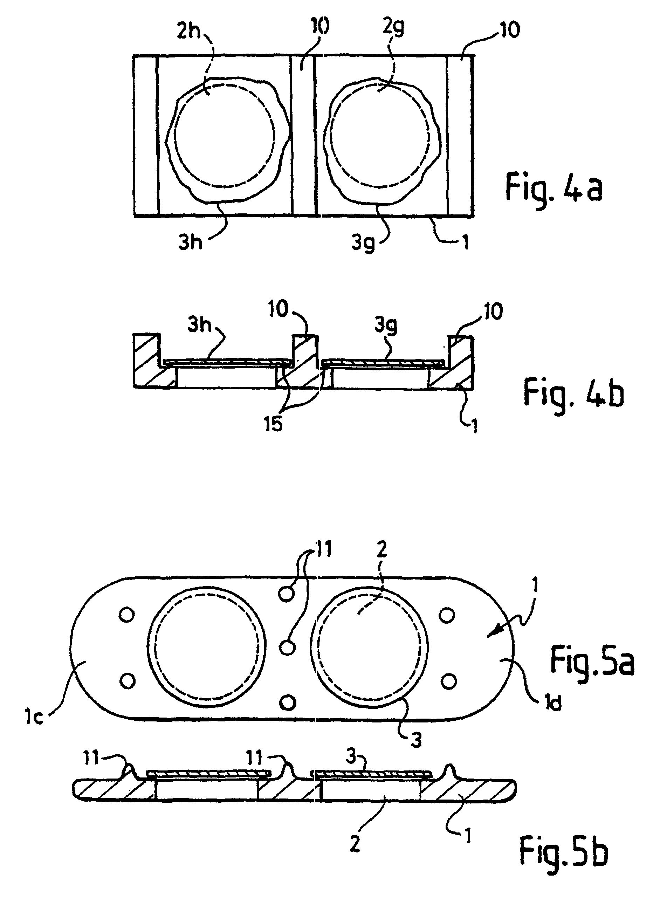

Turning now to figure 4, an alternative embodiment of the test device according to the invention is shown. Features described previously have been accorded corresponding labels.

In this embodiment, supportive material 3 is attached to one side of substrate 1 by fixing means 15. Ridges 10 serve to space sections 3 from contact with external objects so reducing the risk of contamination of the samples carried on sections 3. Ridges 10 may also serve as handling means for the test device for both manual and machine handling.

A test device is shown In figure 5. Projections 11 are used to provide spacing between material 3 and any external surfaces. The distribution of projection 11 may be optimised so that access to material 3 during sample deposition is made easier. Furthermore, in this embodiment, substrate 1 is shown to have an ergonomic shape in that sides 1c and 1d are smoothed and rounded. This adaptation is particularly useful for test devices that are to be used for the collection of, for example, a mid-stream urine sample.

It will be understood from the above that substrate 1 may be of a variety of shapes and forms and that the number, size and shape of first and second apertures 2 may vary according to the requirements of a particular test or tests to be carried out. The above embodiments are merely examples of possible arrangements for the test device. It is envisaged that the number and size of apertures will vary according to the number of tests to be carried out, the number of samples to be stored for future use and the quantity of sample required for any one test.

It will also be understood that different sized and shaped apertures may be provided on a single test device and further said test device may be subject to analysis using more than one type of test.

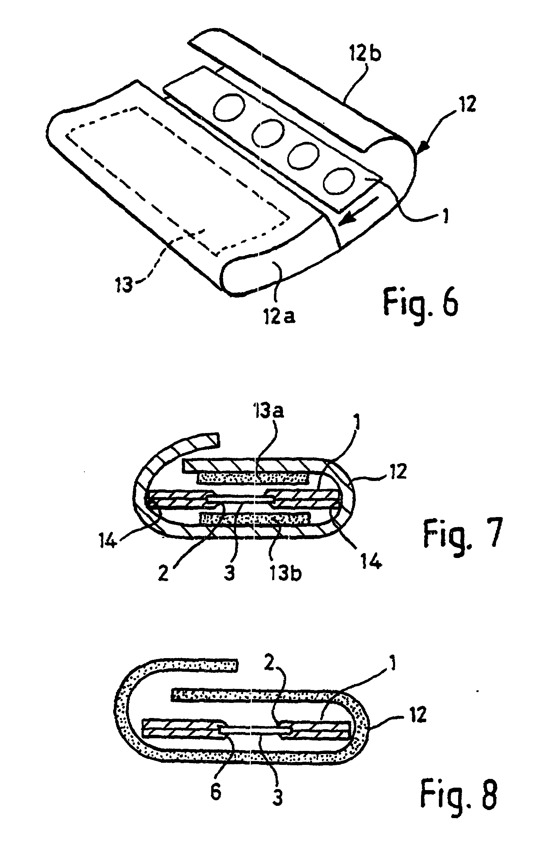

In a further aspect of the invention, shown in figure 6, a carrying pouch 12 is provided. Pouch 12 comprises a receiving portion 12a and a sealing portion 12b. Pouch 12 may be made of, for example, any suitable impervious material.

Test device 1 is inserted into receiving portion 12a, and pouch 12 is subsequently sealed using portion 12b.

Ideally, as shown in figure 7, pouch 12 is so sized and shaped such that test device 1 is securely located inside the pouch by means of edges 14 of test device 1 abutting against the inner sides of pouch 12. In the particular embodiment shown in figures 6 and 7, desiccant layers 13a and 13b are provided on the inner surfaces of pouch 12. The location of layers 13a and 13b is such that following insertion of test device 1, the layers are substantially adjacent first apertures 2 and supportive material sections 3.

Alternatively, layers 13a and 13b may be arranged as aforementioned and embedded in pouch 12.

Alternatively, the sides of pouch 12 may be made almost entirely from a desiccant material such that the entire test device 1 is surrounded by a desiccant layer, see figure 8. In this case, the precise relative geometry of pouch 12 and test device 1 is not so critical since all of device 1 is encompassed within a desiccant atmosphere. However, whatever the preferred arrangement, test device 1 and pouch 12 will normally be designed so that there is little relative movement between them when test device 1 is located within pouch 12.

Silica gel may be used inside layer 13 or the sides of pouch 12 in order to provide a desiccant atmosphere.

In use, a patient will be provided with a kit comprising a test device, a pouch, sample obtaining means and instructions.

In order to obtain, for example, a blood sample, a lance or blade, preferably automatic, will be provided. The usual place from which a patient obtains a sample of his or her own blood is in the fleshy part of the fingers. If this is the chosen site, the hands must be washed and then the lance or blade used to pierce the finger. Once a spot of blood has accumulated, this can be lightly touched to the supportive material, within an aperture of a test device. This is repeated at further locations on the test device until the required number of samples have been obtained.

The test device may then be inserted directly into a pouch 12 as shown in figures 6 - 8 following minimal drying time. The entire arrangement can then be sent, preferably, be post, to an analysis laboratory. The presence of the desiccant within the pouch will ensure that samples are continually exposed to a dry atmosphere during transport to the laboratory so that further evaporation of moisture from the samples is encouraged. Furthermore, the test device ensures that contact between the supportive material impregnated with samples and the inner surfaces of the pouch is not possible.

Alternatively, samples of saliva may be required. In this case, a pipette may be used to obtain a quantity of saliva from the mouth. This is then applied to the supportive material at the required number of locations. The test device may then be sent to an analysis centre.

If a sample of urine is required, the patient has two options. Firstly, he may collect urine in a bottle provided with the test kit and then apply a sample of the urine to the supportive material within a number of apertures. Secondly, and alternatively, or in addition, the test device may be so adapted as to be held adjacent to the urine stream during excretion of urine. In this latter case, it may be advantageous to wait a few moments for excess droplets to fall from the test device before insertion into the pouch and subsequent postage.

Once a test device is received at an analysis laboratory, it is loaded into automatic testing apparatus by a technician. Subsequently, the apparatus, typically, reads the bar code, identifies the type and number of tests to be carried out and the geometry of the test device. In addition, the apparatus locates and punches out, or otherwise removes, the required number of samples from within the corresponding number of apertures.

Fluid samples may be obtained from a variety of different sources, for example, farm animals, household pets, waterways such as rivers, streams and sewage pipes and household pipes and tanks. Subsequent testing, particularly for pipes, tanks and water courses may be directed towards the detection of, for example, particular strains of bacteria as well as evidence of diseases.

It can be seen that use of a test device and carrying pouch according to the invention greatly facilitates the drying, storage, transport and subsequent analysis of fluid samples.

It will be understood from the above that, the use of such test kits comprising a test device and carrying pouch, by, for example, patients in the privacy of their own home and subsequent postage to an analysis laboratory will greatly reduce the demands on the time of professionals in, for example, clinics, surgeries and hospitals. In this way, the cost of mass screening and in particular mass screening of specific subsections of the population is significantly decreased. Indeed, the use of mass screening for a far greater number of diseases and conditions will now be commercially viable. As a result, it can be seen that the current invention provides a significant advance over the prior art.

## Claims

1. A test device for use in automated testing apparatus comprising: a substrate (1) of size and shape suitable for handling by said automated testing apparatus, and including at least one indentation or aperture (2) wherein said indentation or aperture is of a location, size and shape suited to the particular test or tests to be carried out; and further comprising a supportive material (3) mounted on at least a part of said substrate so as to be at least partially positioned over said indentation or aperture; **characterised in that** said supportive material (3) comprises a guide means (14) comprising a sample deposition portion (15a) and attached thereto a channel portion (16) including an indicator means (17); whereby the positioning of a sample to be tested on said sample deposition portion of said supportive material results in said sample travelling along said channel portion and interacting with said indicator means so as to provide a measure of the adequacy of the fluid sample collected.

2. A test device according to Claim 1 wherein said supportive material (3) is spaced from an outer most surface of said substrate (1).

3. A test device according to Claims 1 or 2 wherein said supportive material (3) is sandwiched between two substrates.

4. A test device according to any preceding claim wherein said substrate (1) is provided with a plurality of spaced first indentations or apertures (2a, 2b, 2c, 2d).

5. A test device according to Claim 4 wherein said first indentations or apertures are evenly spaced there apart.

6. A test device according to any preceding claim comprising a holding means (10), whereby the handling of said test device by automated apparatus is facilitated.

7. A test device according to any preceding claim wherein the guide means (14) comprises a hydrophobic material such as latex or wax, which is provided on at least a part of at least one surface of said supportive material.

8. A test device according to Claim 7 wherein said hydrophobic material is latex or wax.

9. A test device according to any preceding claim wherein said substrate is provided with at least one second indentation or aperture (2a, 2h, 2i, 2j) suitably sized and shaped and positioned, with respect to said first aperture, so as to be aligned with said channel portion of said guide means.

10. A test device according to Claim 9 wherein said indentation or aperture is positioned so as to be aligned with said indicator means.

11. A test device according to any preceding claim wherein said indicator means is impregnated with, or cross-linked to, or coated onto, at least a part of at least one surface of said supportive material.

12. A test device according to any preceding claim wherein said sample deposition portion is circular and the diameter of same is greater than the diameter of the first indentation or aperture.

13. A test device according to Claim 12 wherein said sample deposition diameter is greater or in the region of 1 to 5mm.

14. A test device according to any preceding claim wherein said supportive material (3) is absorbent in nature.

15. A test device according to any preceding claim wherein said supportive material (3) comprises a hydrophobic membrane.

16. A test device according to any preceding claim wherein said supportive material (3) is provided with colourmetric and/or fluorometric and/or luminometric and/or radiometric indicator means whereby fluid samples may be analysed.

17. A test device according to any preceding claim wherein said device is provided with identification means.

18. A test kit comprising a combination of the test device according to any preceding claim with a pouch (12) that is of a size and shape that corresponds to the size and shape of said test device.

19. A test kit according to Claim 18 wherein said pouch comprises a desiccant (13a, 13b).

20. A test kit according to Claim 19 wherein said desiccant comprises at least a part of at least one surface of said pouch (12).

21. A test kit according to Claims 19 or 20 wherein said desiccant is provided on an inner surface of said pouch (12).

22. A test kit according to Claims 19 to 21 wherein said pouch comprises a desiccant surface which is so sized and shaped so that when the test device is inserted into the pouch the supportive material contained in the test device is opposite, or adjacent, the desiccant.

23. A test kit according to Claims 19 to 22 wherein said desiccant comprises silica gel.

24. A test kit according to Claims 18 to 23 wherein at least a part of the outer surface of the pouch is made from impervious material.

25. A test kit according to Claims 18 to 24 comprising a means for obtaining a sample.

26. A test kit according to Claim 25 wherein said means for obtaining a sample comprises a lance or blade, if a blood sample is required; a pipette if a saliva sample is required; and/or a container if a urine and/or stool sample is required.

27. A test kit according to Claims 18 to 26 comprising instructions and/or a bar code for identifying purposes.

28. A test kit according to Claim 27 wherein an identification means is provided to indicate the identity and origin of each individual test device, the type of test to be carried out and/or the particular shape of the test device whereby automated testing apparatus can be automatically re-configured following reading of the identification means to accommodate test devices of a variety of shapes and for a variety of tests.

29. A method for confirming the adequacy of a collected fluid sample using the test device according to Claims 1 to 18, comprising;
(i) providing a substrate (1) of a suitable size and shape, and including at least one indentation or aperture (2) wherein said indentation or aperture is of a location, size and shape suited to the particular test or tests to be carried out; and further comprising a supportive material (3) mounted on at least a part of said substrate (1) so as to be at least partially positioned over said indentation or aperture; **characterised in that** said supportive material (3) comprises a guide means (14) comprising a sample deposition portion (15a) and attached thereto a channel portion (16) including an indicator means (17);
(ii) placing a fluid sample on said sample deposition portion (15a) and allowing said fluid sample to fill and/or permeate into said channel portion (16);
(iii) collecting sufficient fluid of said sample so that said sample passes over said indicator means (17) in or associated with said channel portion (16);
(iv) assessing said collected fluid sample by visualisation of said indictor means and/or by automated machine analysis of said indicator means (16).

## Patentansprüche

1. Test-Vorrichtung zur Verwendung in einem automatisierten Testgerät, aufweisend: einen Träger (1) einer Größe und Form, die für die Handhabung in dem automatisierten Testgerät geeignet ist und mindestens eine Vertiefung oder Öffnung (2) einschließt, wobei die Vertiefung oder Öffnung an einer Stelle, von einer Größe und einer Form ist, die für den oder die jeweiligen durchzuführenden Test oder Tests geeignet ist, und ferner aufweisend ein Träger-Material (3), das mindestens auf einem Teil des Trägers befestigt ist, so dass es mindestens teilweise über der Vertiefung oder Öffnung positionierbar ist; **dadurch gekennzeichnet, dass** das Träger-Material (3) ein Führungs-Mittel (14) aufweist; welches einen Proben-Ablagerungs-Abschnitt (15a), und einen daran befestigten Kanal-Abschnitt (16) einschließlich einem Indikator-Mittel (17) aufweist; wobei die Positionierung einer auf dem Proben-Ablagerungs-Abschnitt des Träger-Materials zu testenden Probe in einer Wanderung der Probe entlang des Kanal-Abschnitts und in einer Wechselwirkung mit dem Indikator-Mittel resultiert, um ein Maß für die Angemessenheit der gesammelten Flüssigkeitsprobe zu liefern.

2. Test-Vorrichtung nach Anspruch 1, wobei das Träger-Material (3) von der äußersten Fläche des Trägers (1) beabstandet ist.

3. Test-Vorrichtung nach Anspruch 1 oder 2, wobei das Träger-Material (3) zwischen zwei Trägern eingelegt ist.

4. Test-Vorrichtung nach einem der vorangehenden Ansprüche, wobei der Träger (1) mit einer Vielzahl von ersten, voneinander beabstandeten Vertiefungen oder Öffnungen (2a, 2b, 2c, 2d) versehen ist.

5. Test-Vorrichtung nach Anspruch 4, wobei die ersten Vertiefungen oder Öffnungen voneinander gleichmäßig beabstandet sind.

6. Test-Vorrichtung nach einem der vorangehenden Ansprüche, die ein Halterungs-Mittel (10) aufweist, wodurch die Handhabung der Test-Vorrichtung durch das automatisierte Gerät vereinfacht ist.

7. Test-Vorrichtung nach einem der vorangehenden Ansprüche, wobei das Führungs-Mittel (14) ein hydrophobes Material wie Latex oder Wachs aufweist, welches mindestens auf einem Teil mindestens einer Oberfläche des Träger-Materials vorgesehen ist.

8. Test-Vorrichtung nach Anspruch 7, wobei das hydrophobe Material Latex oder Wachs ist.

9. Test-Vorrichtung nach einem der vorangehenden Ansprüche, wobei der Träger mit mindestens einer zweiten Vertiefung oder Öffnung (2a, 2h, 2i, 2j) versehen ist, die geeignet groß und geformt und positioniert in Bezug auf die erste Öffnung ist, um mit dem Kanal-Abschnitt des Führungs-Mittels verbunden zu werden.

10. Test-Vorrichtung nach Anspruch 9, wobei die Vertiefung oder Öffnung so positioniert ist, um mit dem Indikator-Mittel verbunden zu werden.

11. Test-Vorrichtung nach einem der vorangehenden Ansprüche, wobei das Indikator-Mittel mit mindestens einem Teil mindestens einer Oberfläche des Träger-Materials imprägniert oder quervernetzt oder auf diesem überzogen ist.

12. Test-Vorrichtung nach einem der vorangehenden Ansprüche, wobei der Proben-Ablagerungs-Abschnitt kreisförmig und der Durchmesser von diesem größer als der Durchmesser der ersten Vertiefung oder Öffnung ist.

13. Test-Vorrichtung nach Anspruch 12, wobei der Proben-Ablagerungs-Durchmesser größer als der oder in dem Bereich von 1 bis 5 mm ist.

14. Test-Vorrichtung nach einem der vorangehenden Ansprüche, wobei das Träger-Material (3) von saugfähiger Natur ist.

15. Test-Vorrichtung nach einem der vorangehenden Ansprüche, wobei das Träger-Material (3) eine hydrophobe Membran aufweist.

16. Test-Vorrichtung nach einem der vorangehenden Ansprüche, wobei das Träger-Material (3) mit kolorimetrischen und/oder fluorimetrischen und/oder luminometrischen und/oder radiometrischen Indikator-Mitteln versehen ist, womit die Flüssigkeitsproben analysiert werden können.

17. Test-Vorrichtung nach einem der vorangehenden Ansprüche, wobei die Vorrichtung mit Identifikations-Mitteln versehen ist.

18. Test-Kit aufweisend eine Kombination der Test-Vorrichtung nach einem der vorangehenden Ansprüche mit einen Beutel (12), der von einer Größe und Form entsprechend der Größe und Form der Test-Vorrichtung ist.

19. Test-Kit nach Anspruch 18, wobei der Beutel ein Trockenmittel (13a, 13b) aufweist.

20. Test-Kit nach Anspruch 19, wobei das Trockenmittel mindestens einen Teil von mindestens einer Oberfläche des Beutels (12) umfasst.

21. Test-Kit nach Anspruch 19 oder 20, wobei das Trockenmittel auf der inneren Oberfläche des Beutels (12) vorgesehen ist.

22. Test-Kit nach den Ansprüchen 19 bis 21, wobei der Beutel eine trocknende Oberfläche aufweist, welche so dimensioniert und geformt ist, dass, wenn die Test-Vorrichtung in den Beutel eingefügt wird, das in der Test-Vorrichtung enthaltene Träger-Material gegenüberliegend oder anliegend dem Trocknungsmittel ist.

23. Test-Kit nach den Ansprüchen 19 bis 22, wobei das Trocknungsmittel Silicagel aufweist.

24. Test-Kit nach den Ansprüchen 18 bis 23, wobei mindestens ein Teil der äußeren Oberfläche des Beutels aus undurchlässigem Material ist.

25. Test-Kit nach den Ansprüchen 18 bis 24, aufweisend ein Mittel zur Beschaffung der Probe.

26. Test-Kit nach Anspruch 25, wobei das Mittel zur Beschaffung der Probe eine Lanzette oder eine Klinge aufweist, wenn eine Blutprobe benötigt wird; eine Pipette wenn eine Speichelprobe benötigt wird; und/oder ein Gefäß, wenn eine Urin und/oder Stuhlprobe benötigt wird.

27. Test-Kit nach den Ansprüchen 18 bis 26 aufweisend Anweisungen und/oder einen Strichcode zu Identifizierungszwecken.

28. Test-Kit nach Anspruch 27, wobei ein Identifizierungs-Mittel zur Kennzeichnung der Identität und Herkunft jeder einzelnen Test-Vorrichtung, des auszuführenden Test-Typs und/oder der jeweiligen Form der Test-Vorrichtung vorgesehen ist, wobei das automatisierte Testgerät nach der Ablesung des Identifizierungs-Mittels automatisch re-konfiguriert werden kann, um Test-Vorrichtungen vielfältiger Form und für eine Vielfalt der Tests anzupassen.

29. Verfahren zur Bestätigung der Angemessenheit einer gesammelten Flüssigkeitsprobe, die Test-Vorrichtung nach den Ansprüchen 1 bis 18 verwendend, aufweisend;
(i) Vorsehen eines Trägers (1) einer geeigneten Größe und Form und mindestens eine Vertiefung oder Öffnung (2) einschließend, wobei die Vertiefung oder Öffnung an einer Stelle, von einer Größe und einer Form ist, die für den oder die jeweiligen durchzuführenden Test oder Tests geeignet ist; und ferner aufweisend ein Träger-Material (3), das an mindestens einem Teil des Trägers (1) befestigt ist, so dass es mindestens teilweise über der Vertiefung oder Öffnung positionierbar ist; **dadurch gekennzeichnet, dass** das Träger-Material (3) ein Führungs-Mittel (14) aufweist, welches einen Proben-Ablagerungs-Abschnitt (15a) und einen daran befestigten Kanal-Abschnitt (16) einschließlich einem Indikator-Mittel (17) aufweist;
(ii) Platzieren der Flüssigkeitsprobe auf dem Proben-Ablagerungs-Abschnitt (15a) und Ermöglichen, dass die Flüssigkeitsprobe, den Kanal-Teil (16) füllt und/oder in diesen durchdringt;
(iii) Sammeln ausreichender Flüssigkeit der Probe, so dass die Probe über das Indikator-Mittel (17), das in dem Kanal-Teil (16) vorgesehen oder mit diesem assoziiert ist, tritt;
(iv) Beurteilen der gesammelten Flüssigkeitsprobe durch Visualisieren des Indikator-Mittels und/oder durch automatische maschinelle Analyse des Indikator-Mittels (16).

## Revendications

1. Dispositif de test destiné à être utilisé dans un appareil de test automatique, comprenant : un substrat (1) ayant une taille et une forme qui conviennent à sa manipulation par le dit appareil de test automatique, et incluant au moins une échancrure ou ouverture (2), dans lequel la dite échancrure ou ouverture présente une position, une taille et une forme qui conviennent au test ou aux tests particuliers devant être mis en oeuvre ; et comprenant, en outre, un matériau de support (3) monté sur au moins une partie du dit substrat de manière à se trouver au moins partiellement positionné au-dessus de la dite échancrure ou ouverture ; **caractérisé en ce que** le dit matériau de support (3) comprend un moyen de guidage (14) comprenant une partie de dépôt d'échantillon (15a) et, fixée à elle, une partie de canal (16) incluant un moyen indicateur (17) ; grâce à quoi, le positionnement d'un échantillon devant être testé sur la dite partie de dépôt d'échantillon du dit matériau de support se traduit par le dit échantillon se déplaçant le long de la dite partie de canal et interagissant avec le dit moyen indicateur de façon à donner une mesure de la suffisance de l'échantillon de fluide collecté.

2. Dispositif de test selon la revendication 1, dans lequel le dit matériau de support (3) est espacé d'une surface la plus extérieure du dit substrat (1).

3. Dispositif de test selon la revendication 1 ou 2, dans lequel le dit matériau de support (3) est pris en sandwich entre deux substrats.

4. Dispositif de test selon l'une quelconque des revendications précédentes, dans lequel le dit substrat (1) comporte une pluralité de premières échancrures ou ouvertures espacées (2a, 2b, 2c, 2d).

5. Dispositif de test selon la revendication 4, dans lequel les dites premières échancrures ou ouvertures sont uniformément espacées.

6. Dispositif de test selon l'une quelconque des revendications précédentes, comprenant un moyen de maintien (10), grâce à quoi la manipulation du dit dispositif de test par l'appareil automatique est facilitée.

7. Dispositif de test selon l'une quelconque des revendications précédentes, dans lequel le moyen de guidage (14) comprend un matériau hydrophobe, par exemple du latex ou de la paraffine, qui est disposé sur au moins une partie d'au moins une surface du dit matériau de support.

8. Dispositif de test selon la revendication 7, dans lequel le dit matériau hydrophobe est du latex ou de la paraffine.

9. Dispositif de test selon l'une quelconque des revendications précédentes, dans lequel le dit substrat comporte au moins une seconde échancrure ou ouverture (2a, 2h, 2i, 2j) dont la taille, la forme et la position sont appropriées, par rapport à la dite première ouverture, de manière à se trouver alignée avec la dite partie de canal du dit moyen de guidage.

10. Dispositif de test selon la revendication 9, dans lequel la dite échancrure ou ouverture est positionnée de façon à se trouver alignée avec le dit moyen indicateur.

11. Dispositif de test selon l'une quelconque des revendications précédentes, dans lequel le dit moyen indicateur est imprégné avec, ou est en réticulation avec, ou est revêtu sur, au moins une partie d'au moins une surface du dit matériau de support.

12. Dispositif de test selon l'une quelconque des revendications précédentes, dans lequel la dite partie de dépôt d'échantillon est circulaire et le diamètre de celle-ci est supérieur au diamètre de la première échancrure ou ouverture.

13. Dispositif de test selon la revendication 12, dans lequel le diamètre de la dite partie de dépôt d'échantillon est supérieur ou se trouve dans la zone de 1 à 5 mm.

14. Dispositif de test selon l'une quelconque des revendications précédentes, dans lequel le dit matériau de support (3) est absorbant par nature.

15. Dispositif de test selon l'une quelconque des revendications précédentes, dans lequel le dit matériau de support (3) comprend une membrane hydrophobe.

16. Dispositif de test selon l'une quelconque des revendications précédentes, dans lequel le dit matériau de support (3) comporte un moyen indicateur colorimétrique et/ou fluorométrique et/ou luminométrique et/ou radiométrique, grâce à quoi des échantillons de fluide peuvent être analysés.

17. Dispositif de test selon l'une quelconque des revendications précédentes, dans lequel le dit dispositif comporte des moyens d'identification.

18. Kit de test, comprenant une combinaison du dispositif de test selon l'une quelconque des revendications précédentes avec une poche (12) dont la taille et la forme correspondent à la taille et à la forme du dit dispositif de test.

19. Kit de test selon la revendication 18, dans lequel la dite poche comprend un déshydratant (13a, 13b).

20. Kit de test selon la revendication 19, dans lequel le dit déshydratant comprend au moins une partie d'au moins une surface de la dite poche (12).

21. Kit de test selon la revendication 19 ou 20, dans lequel le dit déshydratant est disposé sur une surface intérieure de la dite poche (12).

22. Kit de test selon les revendications 19 à 21, dans lequel la dite poche comprend une surface déshydratante dont la taille et la forme sont telles que lorsque le dispositif de test est inséré dans la poche, le matériau de support contenu dans le dispositif de test se trouve opposé ou adjacent au déshydratant.

23. Kit de test selon les revendications 19 à 22, dans lequel le dit déshydratant comprend un gel de silice.

24. Kit de test selon les revendications 18 à 23, dans lequel au moins une partie de la surface extérieure de la poche est fabriquée à partir d'un matériau étanche.

25. Kit de test selon les revendications 18 à 24, comprenant un moyen destiné à obtenir un échantillon.

26. Kit de test selon la revendication 25, dans lequel le dit moyen destiné à obtenir un échantillon comprend une lancette ou une lame, si un échantillon sanguin est requis ; une pipette, si un échantillon de salive est requis ; et/ou un conteneur, si un échantillon d'urine et/ou de selle est nécessaire.

27. Kit de test selon les revendications 18 à 26, comprenant des instructions et/ou un code barre destiné à des fins d'identification.

28. Kit de test selon la revendication 27, dans lequel un moyen d'identification est prévu pour indiquer l'identité et l'origine de chaque dispositif de test individuel, le type de test devant être mis en oeuvre et/ou la forme particulière du dispositif de test, grâce à quoi l'appareil de test automatique peut être automatiquement reconfiguré après qu'aient été lus les moyens d'identification pour adapter les dispositifs de test à diverses formes et à divers tests.

29. Procédé pour confirmer la suffisance d'un échantillon de fluide collecté en utilisant le dispositif de test selon les revendications 1 à 18, comprenant les étapes consistant :
(i) à mettre en place un substrat (1) ayant une taille et une forme appropriées, et incluant au moins une échancrure ou ouverture (2), dans lequel la dite échancrure ou ouverture présente une position, une taille et une forme qui conviennent au test ou aux tests particuliers devant être mis en oeuvre ; et comprenant, en outre, un matériau de support (3) monté sur au moins une partie du dit substrat (1) de manière à se trouver au moins partiellement positionné au-dessus de la dite échancrure ou ouverture ; **caractérisé en ce que** le matériau (3) comprend un moyen de guidage (14) comprenant une partie de dépôt d'échantillon (15a) et, fixée à elle, une partie de canal (16) incluant un moyen indicateur (17) ;
(ii) à placer un échantillon de fluide sur la dite partie de dépôt d'échantillon (15a) et à laisser le dit échantillon de fluide remplir et/ou s'infiltrer dans la dite partie de canal (16) ;
(iii) à collecter un fluide suffisant du dit échantillon de telle sorte que le dit échantillon passe par-dessus du dit moyen indicateur (17) dans ou en association avec la dite partie de canal (16) ;
(iv) à estimer Le dit échantillon de fluide collecté par visualisation du dit moyen indicateur et/ou par l'analyse au moyen d'une machine automatique du dit moyen indicateur (16).
